Europäisches Patentamt

European Patent Office

Office européen des brevets

(19)

(11)  EP 0 714 656 B1

(12)  **EUROPEAN PATENT SPECIFICATION**

(45) Date of publication and mention
of the grant of the patent:
**28.02.2001  Bulletin 2001/09**

(51) Int Cl.[7]: **A61K 9/48**

(21) Application number: **95307898.7**

(22) Date of filing: **06.11.1995**

(54) **Capsule shell compositions and their use**

Zusammensetzungen zum Herstellen von Kapselhüllen und deren Anwendung

Compositions pour préparer des enveloppes de capsules et leur utilisation

(84) Designated Contracting States:
**DE ES FR GB IT**

(30) Priority: **01.12.1994  JP  32358194**
**16.12.1994  JP  33396594**

(43) Date of publication of application:
**05.06.1996  Bulletin 1996/23**

(73) Proprietor: **Shionogi Qualicaps Co., Ltd.**
**Yamatokoriyama-shi, Nara-ken (JP)**

(72) Inventors:
• **Yamamoto, Taizo**
**Osaka-shi, Osaka (JP)**
• **Matsuura, Seinosuke**
**Souraku-gun, Kyoto-fu (JP)**
• **Akai, Kazukiyo**
**Kashihara-shi, Nara-ken (JP)**

(74) Representative: **Stoner, Gerard Patrick et al**
**MEWBURN ELLIS**
**York House**
**23 Kingsway**
**London WC2B 6HP (GB)**

(56) References cited:
**EP-A- 0 592 130**          **DE-A- 2 029 402**
**US-A- 5 264 223**

EP 0 714 656 B1

## Description

This invention relates to a composition for forming medical hard capsules. More particularly, it relates to such a capsule shell composition using hydroxypropylmethyl cellulose as a base.

Medical hard capsules are conventionally formed from compositions comprising gelatin as a base with a plasticizer such as glycerin and sorbitol, opaque agent, dye, pigment and other additives blended therein. After molding pins are immersed in a gelatin aqueous solution with such components blended and withdrawn therefrom, the gelatin solution adhering to the pins is dried, obtaining capsule shells.

The shell-forming compositions based on gelatin have the problem that the plasticity and other properties of shells largely depend on a water content. With a too low water content, shells are less resistant against shocks as encountered on drug filling. Also, as the water content lowers due to drying during shelf storage, shells can contract to loosen the cap-body engagement of capsules.

For gelatin capsules, it is thus critical to maintain the water content constant. However, since the optimum water content is as high as about 10 to 15% by weight, there is a likelihood that the water in the capsule shell can affect the drug fill to lower its titer, degrade its quality, and change its color and conversely, the capsule shell can be insolubilized if the drug fill is susceptible to hydrolysis or is a mixture of interacting ingredients. Therefore, there is a demand to have capsules based on a substance other than gelatin so that the material of capsules can be selected in accordance with a particular drug fill.

Medical capsules using a base other than gelatin are known in the art. Typically, capsules based on water-soluble cellulose derivatives were proposed. For example, Japanese Patent Publication (JP-B) No. 4310/1972 discloses a method for preparing capsules based on water-soluble cellulose ether from an aqueous solution of water-soluble cellulose ether. Japanese Patent Application Kokai (JP-A) Nos. 100519/1986 and 266060/1987 disclose to prepare capsules from an aqueous solution of water-soluble cellulose ether and polyvinyl alcohol (PVA) blended therewith.

JP-B-4310/1972's shell forming method involves the steps of immersing molding pins in an aqueous solution of water-soluble cellulose derivative and heating the pins and hence, the coating adhered thereto for gelation. The coating is not gelled or solidified and can fall down from the pins if heating is insufficient. The coating can be wrinkled during gelation if the heating temperature is too high. In the latter method of preparing capsules from an aqueous solution of water-soluble cellulose derivative and PVA, the water-soluble cellulose derivative adhered to the molding pins is gelled by immersing it in hot water. Some of the gelled coating can be dissolved in the hot water at this point, hindering formation of uniform shells. In addition, due to low jelly strength, the dried shells can often be cracked upon removal from the molding pins. In either of these methods, it is difficult to produce capsule shells having a low water content.

Additionally, these methods require a special apparatus or operation of heating the molding pins or immersing the molding pins with cellulose coating in hot water. Unfortunately, it is impossible to utilize the current manufacturing apparatus for gelatin capsules without a substantial change.

To solve these problems, the applicant previously proposed in US-A-5,264,223 a medical hard capsule having a low water content which is shaped from a capsule shell composition comprising a water-soluble cellulose derivative as a base, a gelling agent and a co-gelling agent. This capsule has equivalent performance to conventional gelatin capsules and can be produced by utilizing the current manufacturing apparatus for gelatin capsules as such.

However, through the continuing research works of the inventors, it was found that this capsule is inferior to conventional gelatin capsules in solubility or disintegrating ability under certain conditions. More particularly, one preferred formulation of this capsule shell composition uses hydroxypropylmethyl cellulose as a water-soluble cellulose derivative base, carrageenan as a gelling agent and a potassium ion as a co-gelling agent. Shells of this preferred formulation take a long time to disintegrate under special conditions where calcium ions are present. Then, if a capsule of this composition filled with drugs is administered after having a food or beverage containing much calcium ions, for example, milk, then the capsule is retarded from disintegration. Then the drugs are not fully released or absorbed within a proper time, failing to fully exert their pharmaceutical effect. Therefore, it is desired to further improve the properties of the capsule based on a water-soluble cellulose derivative.

The general aim herein is to provide new and useful capsule shell compositions, their use, and the resulting shells; also the shells when loaded with pharmaceutical.

One preferred aim herein is to provide a capsule shell composition based on a water-soluble cellulose derivative which does not degrade its disintegration ability under special conditions where much calcium ion is present, that is, exerts its performance under any condition.

In connection with the capsule shell composition comprising hydroxypropylmethyl cellulose (to be abbreviated as HPMC, hereinafter) as a water-soluble cellulose derivative base, carrageenan as a gelling agent, and a potassium ion as a co-gelling agent wherein the shapability of HPMC is improved by blending carrageenan as a gelling agent and gelling this carrageenan with the co-gelling agent, we found that the disintegration ability of this composition is degraded in the presence of calcium ions because the calcium ions inhibit dissolution of the carrageenan blended in the composition as the gelling agent.

Continuing research work , we have found that degradation of the disintegration ability due to the presence of calcium ions is restrained by using a larger proportion of a HPMC having a relatively low viscosity as a base, increasing the amount of the co-gelling agent blended, and minimizing the proportion of carrageenan gelling agent within a sufficient range to insure good shapability. More particularly, by using a HPMC having a viscosity of 2.4 to 5.4* $10^{-6}$ $\frac{m^2}{s}$ (2.4 to 5.4 centistokes) as measured in a 2% aqueous solution at 20°C, and blending 18 to 28 parts by weight of the HPMC with 0.01 to 0.1 part by weight of carrageenan as a gelling agent and 0.05 to 0.6 part by weight of a co-gelling agent, we can obtain a capsule shell composition which maintains satisfactory disintegration ability even in the presence of calcium ions and exerts performance comparable to conventional gelatin capsules.

Accordingly, the present invention provides a capsule shell composition comprising

18 to 28 parts by weight of a hydroxypropylmethyl cellulose, having a viscosity of 2.4 to 5.4* $10^{-6}$ $\frac{m^2}{s}$ (2.4 to 5.4 centistokes) as measured in a 2% aqueous solution at 20°, as a base,
0.01 to 0.1 part by weight of carrageenan as a gelling agent, and
0.05 to 0.6 part by weight of at least one ion of potassium and calcium ions as a co-gelling agent.

BRIEF DESCRIPTION OF THE DRAWINGS

FIG. 1 schematically illustrates the gelation mechanism of carrageenan.
FIG. 2 is a graph showing the percent leaching of the contents from a capsule of Example 1 and a conventional gelatin capsule when they were immersed in a first solution prescribed in the Pharmacopoeia of Japan.
FIG. 3 is a graph showing the percent leaching of the contents from a capsule of Example 1 and a conventional gelatin capsule when they were immersed in a second solution prescribed in the Pharmacopoeia of Japan.

FURTHER EXPLANATIONS; PREFERRED AND OPTIONAL FEATURES

In a capsule shell composition comprising HPMC as a base, carrageenan as a gelling agent and a co-gelling agent for assisting in gelation of carrageenan, the present invention optimizes the viscosity of HPMC and the blending proportion of the respective components such that the composition may maintain satisfactory disintegration ability even under special conditions where many calcium ions are present.
The HPMC used as the base may be a commercially available powder product. According to the invention, the HPMC should be a low viscosity one such that a 2% aqueous solution of HPMC has a viscosity of 2.4 to 5.4*$10^{-6}$ $\frac{m^2}{s}$ (2.4 to 5.4 centistokes) at 20°C, preferably 3.0 to 4.6*$10^{-6}$ $\frac{m^2}{s}$ (3.0 to 4.6 centistokes) at 20°C. As defined herein, the viscosity of HPMC is not the viscosity of HPMC itself, but the viscosity of a 2% aqueous solution of HPMC throughout the specification. With a viscosity of less than 2.4*$10^{-6}\frac{m^2}{s}$(2.4 centistokes), an immersion solution of HPMC from which a capsule shell composition is to be obtained by a dipping technique has a viscosity too low to shape the capsule shell composition. With a viscosity of more than 15.4*$10^{-6}\frac{m^2}{s}$( 5.4 centistokes), an immersion solution has too high a viscosity, which requires to reduce the amount of HPMC blended which in turn, requires to increase the proportion of the gelling agent blended, failing to achieve the desired aim.
Such low viscosity HPMC is commercially available e.g. as TC-5M type HPMC (2% aqueous solution viscosity 4.5*$10^{-6}$ $\frac{m^2}{s}$(4.5 centistokes) at 20°C) and TC-5E type HPMC (2% aqueous solution viscosity 3.0*$10^{-6}$ $\frac{m^2}{s}$(3.0 centistokes) at 20°C) from Shin-Etsu Chemical Co., Ltd. These HPMC products may be used alone or suitably blended to form a mixture having a viscosity of 3.0 to 4.6*$10^{-6}$ $\frac{m^2}{s}$ (3.0 to 4.6 centistokes). Alternatively, such a HPMC product may be blended with another HPMC product having higher or lower viscosity (by itself outside the range prescribed here) to form a mixture having an optimum viscosity as defined- above.
The proportion of HPMC blended is 18 to 28 parts by weight, preferably 19 to 25 parts by weight relative to the remaining components to be defined later. Several inconvenient problems occur if the proportion of HPMC blended is outside this range. Capsule shells may be formed from the capsule shell composition according to the invention by dissolving the composition in water to form an aqueous immersion solution, immersing molding pins in the immersion solution, withdrawing the pins from the solution with the solution adhering to the periphery of the pins, and drying the adhering solution. If the proportion of HPMC blended is less than 18 parts by weight, the proportion of the gelling agent blended becomes relatively high, failing to achieve the desired aim herein. If the proportion of HPMC blended is more than 28 parts by weight, the proportion of the gelling agent blended becomes relatively low, but the immersion solution has a viscosity too high to shape capsule shells by the dipping technique.
Carrageenan is blended as the gelling agent. Carrageenan generally includes three types, iota ($\iota$), kappa ($\kappa$) and lambda ($\lambda$). Among these, $\iota$-carrageenan and $\kappa$-carrageenan having a gelling ability are suitable, with the K-carrageenan being preferred.
The proportion of carrageenan blended is 0.01 to 0.1 part by weight, preferably 0.01 to 0.09 part by weight, more preferably 0.07 to 0.09 part by weight relative to the above-mentioned amount of HPMC. If the proportion of carrageenan blended is less than 0.01 part by weight, no satisfactory degree of gelation is achieved and shells of sufficient gage cannot be formed by the dipping technique. If the proportion of carrageenan blended exceeds 0.1 part by weight, the composition loses disintegration ability in the presence of calcium ions.

The co-gelling agent for assisting in gelation of carrageenan comprising potassium ion, calcium ion or both. As a preferred rule, calcium ion is used for $\iota$-carrageenan and potassium ion is used for κ-carrageenan. It is most preferred to use κ-carrageenan as the gelling agent and a potassium ion as the co-gelling agent. The potassium ion may be blended in the form of a water-soluble compound such as potassium chloride, potassium phosphate and potassium citrate. The calcium ion may also be blended in the form of a water-soluble compound such as calcium chloride.

The proportion of co-gelling agent blended is 0.05 to 0.6 part by weight, preferably 0.06 to 0.1 part by weight in ionic amount relative to the above-mentioned amount of HPMC and gelling agent. If the proportion of the agent blended is less than 0.05 part by weight, no satisfactory gelation of carrageenan is achieved and shells of sufficient gage cannot be formed by the dipping technique. If the proportion of co-gelling agent blended exceeds 0.6 part by weight, a gelled film forms in an aqueous immersion solution, shell formation by the dipping technique is difficult, and shells, even formed, are low in disintegration ability.

Though the invention is not bound to the theory, the reason why the capsule shell composition of the invention maintains satisfactory disintegration ability even in the presence of calcium ions is thought to be as follows.

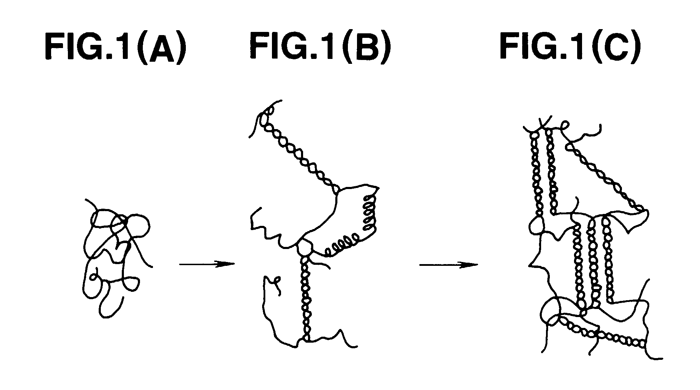

As mentioned above, the shapability of HPMC is improved by gelling carrageenan as the gelling agent with the co-gelling agent. The gelation of carrageenan follows the mechanism schematically shown in FIG.1, i.e carrageenan molecules form double helix structures with the aid of the co-gelling agent (FIG. 1B) to form a three-dimensional network. If the thus gelled carrageenan comes in contact with calcium ions, the double helix structures increase to strengthen the three-dimensional network (FIG. 1C). Also crosslinking occurs between adjacent sulfate groups in adjacent double helix structures to stabilize the three-dimensional network. Then the gel increases its hardness to detract from solubility or disintegration ability. However, in the composition of the present invention, by using a HPMC having a relatively low viscosity as a base, increasing the proportion of HPMC used, increasing the amount of the co-gelling agent blended, and minimizing the proportion of carrageenan gelling agent within a sufficient range to insure good shapability, the amount of carrageenan relative to HPMC is set at a very low level. Then even when double helix structures of carrageenan increase upon contact with calcium ions and crosslinking occurs between sulfate groups in double helix structures, the interstice structure resulting from tangling of carrageenan molecules is maintained in a relatively coarse state so that strong gelation does not occur. In this way, satisfactory disintegration ability is maintained.

We find that such a capsule shell composition containing the above-defined HPMC base, carrageenan gelling agent and co-gelling agent in the above-defined proportion exhibits satisfactory disintegration ability even in an environment where calcium ions are present. In one preferred embodiment, the composition forms a capsule shell of 0.1 mm thick which will have an opening time within 4 minutes, more preferably within 2-1/2 minutes when immersed in an aqueous solution of 0.1M potassium chloride at 37°C. The potassium ion inhibits dissolution of carrageenan through a similar mechanism to the inhibitory mechanism of the calcium ion and rather to a greater extent than the calcium ion. The dissolution of a shell in the presence of potassium ions can represent the dissolution of a shell in the presence of calcium ions. A shell having satisfactory dissolution characteristics in an aqueous solution of potassium chloride will exhibit satisfactory disintegration ability comparable to that of conventional gelatin shells even in an environment where much calcium ion is present. It is thus understood that a shell composition as described having a dissolution time as defined above of more than 4 minutes will still have practically acceptable dissolution property in the presence of calcium ions.

In the capsule shell composition of the invention containing the above-defined HPMC base, carrageenan gelling agent and co-gelling agent in the above-defined proportion, there may be further blended various additives such as coloring agents (e.g., dyes and pigments), opaque agents, and flavors in conventional amounts. It is noted that the capsule shell composition of the invention desirably has a water content of about 3 to 6% by weight, ensuring formation of hard capsules with a low water content.

The capsule shell composition may be prepared in the form of a capsule shell by a well-known dipping technique as used in the manufacture of conventional gelatin capsule shells. More particularly, medical hard capsules are prepared by blending the HPMC, gelling agent, co-gelling agent and optional additives in water to form an aqueous solution or immersion solution, once immersing molding pins in the immersion solution, withdrawing the pins from the solution with the solution adhering to the periphery of the pins, drying the adhering solution to form capsule shells (body or cap), and removing the shells from the pins. The shells are cut to a suitable size if necessary. A pair of body and cap shells are mated to form a capsule. In this way, the capsule shell composition of the invention is available in the form of a capsule shell.

In shaping the capsule shell composition by the above-mentioned dipping technique, the immersion solution in which shaping pins are immersed is preferably set at a temperature of 48 to 55°C, especially 51 to 53°C. Outside this temperature range, the immersion solution would have a finely varying jelly viscosity and thickly or thinly adhere to the pins, failing to form shells of uniform gage. Thereafter the immersion solution adhering to the pins is preferably dried at a temperature of 25 to 35°C for 40 to 60 minutes. Through the drying step, the immersion solution adhering to the pins is concentrated to form hard shells around the pins. Other conditions may be the same as used in the manufacture of conventional gelatin shells.

Hard capsules formed of the present compositions are well suited for medical application. They may be also used in other applications such as food.

We find that these capsule shell compositions have good shapability and disintegration ability comparable to conventional gelatin shells even in special conditions where much calcium ion is present. HPMC capsules of the inventive composition will effectively disintegrate in the stomach even when they are administered after drinking milk containing much calcium ions, achieving equivalent performance to conventional gelatin capsules. Then the invention enables to take full advantage of HPMC-base hard capsules.

EXAMPLE

Examples of the invention are given below by way of illustration and not by way of limitation. All percents are by weight.

Example 1 and Comparative Example 1

Potassium chloride was dissolved in pure water at about 75°C. With stirring, κ-carrageenan and a coloring agent (titanium oxide) were added to the solution and dissolved therein. With stirring, hydroxypropylmethyl cellulose (HPMC) was added to the solution and dispersed therein. The solution was cooled to a temperature of 50°C and further agitated for dissolving the HPMC therein. The solution was then allowed to stand for deaeration. In this way, two immersion solutions were obtained as shown in Table 1.

A conventional capsule shell forming apparatus was charged with the immersion solution which was maintained at 52°C. The apparatus was operated in accordance with a conventional dipping technique to prepare No. 2 capsule shells of the shell composition shown in Table 1 having a thickness of 0.1 mm. In this way, two types of capsule shells were obtained.

Table 1

|  | Example 1 | Comparative Example 1 |
|---|---|---|
| Immersion solution |  |  |
| HPMC: TC-5R | - | 16% |
| TC-5MW | 10% | - |
| TC-5EW | 10% | - |
| Viscosity $3.8*10^{-6} \frac{m^2}{s}$ | (3.8 cst) $6.0 \cdot 10^{-6} \frac{m^2}{s}$ | (6.0 cst) |
| κ-carrageenan | 0.08% | 0.2% |
| Potassium chloride | 0.11% | 0.1% |
| (potassium ion) | (0.06%) | (0.05%) |
| Titanium oxide | 0.77% | 0.62% |
| Capsule shell |  |  |
| HPMC | 90.63% | 89.83% |
| κ-carrageenan | 0.36% | 1.12% |
| Potassium chloride | 0.50% | 0.56% |
| (potassium ion) | (0.26%) | (0.29%) |
| Titanium oxide | 3.51% | 3.49% |

Note: TC-5R, TC-5MW and TC-5EW, as mentioned in this application, are trade names of HPMC manufactured by Shin-Etsu Chemical Co., Ltd. TC-5R has a viscosity of 6.0 centistokes; TC-5MW has a viscosity of $4.5 \cdot 10^{-6} \frac{m^2}{s}$ (4.5 centistokes); and TC-5EW has a viscosity of $3.0 \ 10^{-6} \frac{m^2}{s}$ (3.0 centistokes), as measured in 2% aqueous solution at 20°C. The viscosity of Example 1 is that of a 1/1 mixture of TC-5MW and TC-5EW.

The capsules were filled with 0.3 g of corn starch and immersed in an aqueous solution of 0.1M potassium chloride at 37°C. The opening time was measured by means of a disintegration tester as prescribed in the Pharmacopoeia of Japan. Three measurements were taken and an average was calculated. The results are shown in Table 2. As a reference, a conventional gelatin capsule was similarly measured for opening time, with the results shown in Table 2.

Table 2

| Capsule | Opening time (min.) | | | |
|---|---|---|---|---|
| Gelatin | 1.3 | 1.5 | 1.5 | av. 1.4 |
| Comparative Example 1 | 4.2 | 4.5 | 6.8 | av. 5.2 |
| Example 1 | 1.9 | 2.2 | 2.4 | av. 2.2 |

Separately, the capsules were filled with 0.7 g of copper wire as a weight and immersed in milk at 37°C. The opening time was measured by means of a disintegration tester as prescribed in the Pharmacopoeia of Japan. Three measurements were taken and an average was calculated. The results are shown in Table 3. As a reference, a conventional gelatin capsule was similarly measured for opening time, with the results shown in Table 3.

Table 3

| Capsule | Opening time (min.) | | | |
|---|---|---|---|---|
| Gelatin | 2 | 3 | 3 | av. 2.7 |
| Comparative Example 1 | 15 | 16 | 18 | av. 16.3 |
| Example 1 | 3 | 3 | 4 | av. 3.3 |

It is seen from Table 3 that the capsule shell composition of the invention exhibits disintegration ability comparable to the conventional gelatin capsule even in milk, containing much calcium ion.

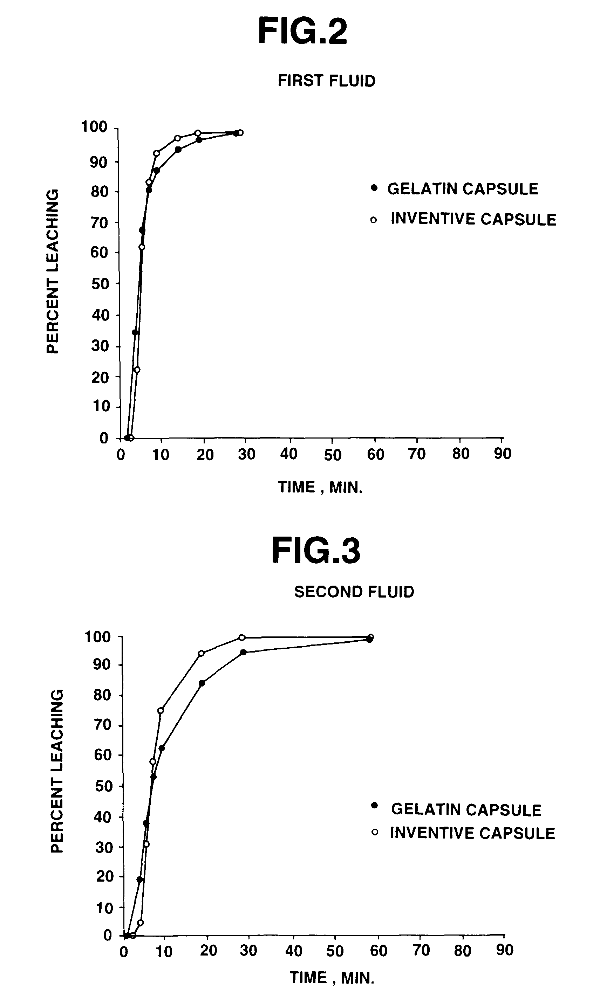

Next, the capsule of Example 1 and a conventional gelatin capsule were evaluated for disintegration ability in the first and second fluids prescribed in the Pharmacopoeia of Japan, Section 12. The capsules were each filled with 300 mg of a mixture of 20 parts by weight of acetaminophen and 280 parts by weight of corn starch. The capsules were immersed in the first and second fluids. While stirring the test solution by rotating a paddle at 100 rpm, the percent leaching of the contents was measured. The results are plotted in the graphs of FIGS. 2 and 3.

It is seen from FIGS. 2 and 3 that the capsule of Example 1 has an equivalent disintegration ability to that of the conventional gelatin capsule in both the first and second fluids prescribed in the Pharmacopoeia of Japan. This suggests that the hard capsules of the shell composition according to the invention are useful as medical capsules.

Examples 2 and 3

Capsule shells were prepared from an immersion solution by the same procedure as in Example 1. The compositions of immersion solutions and capsule shells are shown in Table 4.

Table 4

| | Example 2 | Example 3 |
|---|---|---|
| Immersion solution | | |
| HPMC: TC-5MW | 18% | - |
| T-C-5EW | - | 28% |
| Viscosity | 4.5 cst | 3.0 cst |
| κ-carrageenan | 0.1% | 0.01% |
| Potassium chloride | 0.11% | 1.0% |
| (potassium ion) | (0.06%) | (0.5%) |
| Titanium oxide | 0.69% | 1.11% |
| Capsule shell | | |
| HPMC | 90.48% | 88.31% |
| κ-carrageenan | 0.50% | 0.03% |
| Potassium chloride | 0.55% | 3.15% |
| (potassium ion) | (0.29%) | (1.65%) |
| Titanium oxide | 3.47% | 3.51% |

Japanese Patent Application No. 333965/1994 is incorporated herein by reference.
Although some preferred embodiments have been described, many modifications and variations may be made thereto in the light of the above teachings. It is therefore to be understood that within the scope of the general teachings herein, the invention may be practised other than as described in the Examples.

## Claims

1.  A capsule shell composition comprising

    18 to 28 parts by weight of hydroxypropylmethyl cellulose base, having a viscosity of 2.4 to 5.4 x $10^{-6}$ $m^2$/s (2.4 to 5.4 centistokes) as measured in a 2% aqueous solution at 20°C;
    0.01 to 0.1 part by weight of carrageenan as gelling agent, and
    0.05 to 0.6 part by weight of potassium ion, calcium ion or both as co-gelling agent.

2.  A composition according to claim 1 in which the hydroxypropylmethyl cellulose has a viscosity, measured in a 2% aqueous solution at 20°C, from 3.0 to 4.6 x $10^{-6}$ $m^2$/s (3.0 to 4.6 centistokes).

3.  A composition according to claim 1 or claim 2 in which the proportion of the hydroxypropylmethyl cellulose base is from 19 to 25 parts by weight.

4.  A composition according to any one of the preceding claims in which the proportion of said co-gelling agent ion(s) is from 0.06 to 0.1 parts by weight.

5.  A composition according to any one of the preceding claims having a water content of from 3 to 6 wt%.

6.  A composition according to any one of the preceding claims wherein said carrageenan gelling agent is κ-carrageenan and the co-gelling agent is potassium ion.

7.  A composition according to any one of the preceding claims which is formable into a capsule shell 0.1 mm thick, having an opening time of less than 4 minutes when immersed in 0.1 M aqueous potassium chloride at 37°C.

8.  A capsule shell formed of a composition according to any one of claims 1 to 7.

9.  A pharmaceutical capsule comprising a pharmaceutical enclosed in a capsule shell according to claim 8.

10. Use of a composition according to any one of claims 1 to 7 to make capsule shells.

## Patentansprüche

1.  Kapselhüllen-Zusammensetzung, umfassend 18 bis 28 Gewichtsteile Hydroxypropylmethylcellulose-Basis mit einer Viskosität von 2,4 bis 5,4 × $10^{-6}$ $m^2$/s (2,4 bis 5,4 Centistokes), gemessen in einer 2%igen wässrigen Lösung bei 20 °C;

    0,01 bis 0,1 Gewichtsteile Carragheen als Geliermittel, sowie
    0,05 bis 0,6 Gewichtsteile an Kaliumionen, Kalziumionen oder beiden als Co-Geliermittel.

2.  Zusammensetzung nach Anspruch 1, bei der die Hydroxypropylmethylcellulose eine Viskosität, gemessen in einer 2%igen wässrigen Lösung bei 20 °C, von 3,0 bis 4,6 × $10^{-6}$ $m^2$/s (3,0 bis 4,6 Centistokes) aufweist.

3.  Zusammensetzung nach Anspruch 1 oder 2, bei der der Anteil der Hydroxypropylmethylcellulosebasis von 19 bis 25 Gewichtsteile beträgt.

4.  Zusammensetzung nach einem der vorangegangenen Ansprüche, bei der der Anteil des/der Co-Geliermittel-Ions bzw. -Ionen 0,06 bis 0,1 Gewichtsteile beträgt.

5.  Zusammensetzung nach einem der vorangegangenen Ansprüche mit einem Wassergehalt von 3 bis 6 Gew.-%.

**6.** Zusammensetzung nach einem der vorangegangenen Ansprüche, worin das Carragheen-Geliermittel κ-Carragheen ist und das Co-Geliermittel das Kaliumion ist.

**7.** Zusammensetzung nach einem der vorangegangenen Ansprüche, die zu einer Kapselhülle mit einer Dicke von 0,1 mm formbar ist, die eine Öffnungszeit von weniger als 4 min aufweist, wenn sie in 0,1 M wässrige Kaliumchlorid-Lösung mit 37 °C eingetaucht wird.

**8.** Aus eine Zusammensetzung nach einem der Ansprüche 1 bis 7 gebildete Kapselhülle.

**9.** Pharmazeutikum-Kapsel, die ein in einer Kapselhülle nach Anspruch 8 eingeschlossenes Pharmazeutikum umfasst.

**10.** Verwendung einer Zusammensetzung nach einem der Ansprüche 1 bis 7 zur Herstellung von Kapselhüllen.

**Revendications**

**1.** Une composition pour capsule comprenant 18 à 28 parties en poids de base d'hydroxypropylméthylcellulose, ayant une viscosité de 2,4 à $5,4 \times 10^{-6}$ m$^2$/s (2,4 à 5,4 centistokes) comme mesuré dans une solution aqueuse à 2 % à 20°C ;

0,01 à 0,1 partie en poids de carragheenan comme agent gélifiant, et
0,05 à 0,1 partie en poids d'ions potassium, calcium ou les deux comme agent co-gélifiant.

**2.** Une composition selon la revendication 1 dans laquelle l'hydroxypropylméthylcellulose a une viscosité, mesurée dans une solution aqueuse à 2 % à 20°C, de 3,0 à $4,6 \times 10^{-6}$ m$^2$/s (3,0 à 4,6 centistokes).

**3.** Une composition selon la revendication 1 ou la revendication 2 dans laquelle la proportion de la base d'hydroxypropylméthylcellulose est de 19 à 25 parties en poids.

**4.** Une composition selon l'une quelconque des revendications précédentes dans laquelle la proportion dudit/desdits ion(s) agent(s) co-gélifiant(s) est de 0,06 à 0,1 partie en poids.

**5.** Une composition selon l'une quelconque des revendications précédentes ayant un contenu en eau de l'ordre de 3 à 6 % en poids.

**6.** Une composition selon l'une quelconque des revendications précédentes où ledit agent gélifiant de carragheenan est du κ-carragheenan et l'agent co-gélifiant est l'ion potassium.

**7.** Une composition selon l'une quelconque des revendications précédentes qui est formable en une enveloppe de capsule de 0,1 mm d'épaisseur, ayant un temps d'ouverture inférieur à 4 minutes lorsqu'elle est immergée dans une solution aqueuse de chlorure de potassium 0,1 M à 37°C.

**8.** Une enveloppe de capsule formée d'une composition selon l'une des revendications 1 à 7.

**9.** Une capsule pharmaceutique comprenant un produit pharmaceutique inclus dans une enveloppe de capsule selon la revendication 8.

**10.** Utilisation d'une composition selon l'une quelconque des revendications 1 à 7 pour faire des enveloppes de capsules.

# FIG.1(A)    FIG.1(B)    FIG.1(C)

# FIG.2

FIRST FLUID

- GELATIN CAPSULE
- INVENTIVE CAPSULE

# FIG.3

SECOND FLUID

- GELATIN CAPSULE
- INVENTIVE CAPSULE